# EUROPEAN PATENT SPECIFICATION

(11) **EP 3 171 715 B1**
(45) Date of publication and mention of the grant of the patent: **06.02.2019**
(21) Application number: 15747243.2
(22) Date of filing: 22.07.2015
(51) Int. Cl.: A23L 33/125, A23L 33/22, A61K 31/702, A61K 31/733, A61P 11/06, A61P 37/08

(54) **PREVENTION OR TREATMENT OF FOOD ALLERGY IN INFANTS AND TODDLERS**
PRÄVENTION ODER BEHANDLUNG VON NAHRUNGSMITTELALLERGIEN IN SÄUGLINGEN UND KLEINKINDERN
PRÉVENTION OU TRAITEMENT DE L'ALLERGIE ALIMENTAIRE CHEZ LES NOURRISSONS ET LES JEUNES ENFANTS

(30) Priority: 24.07.2014 WO PCT/NL2014/050507
(43) Date of publication of application: 31.05.2017
(73) Proprietor: N.V. Nutricia, 2712 HM Zoetermeer (NL)
(72) Inventor: JEURINK, Prescilla, NL-3584 CT Utrecht (NL); LOBATO-VAN ESCH, Betty, NL-3584 CT Utrecht (NL)
(74) Representative: Nederlandsch Octrooibureau
(86) International application number: PCT/NL2015/050537
(87) International publication number: WO 2016/013935

(56) References cited:
- EP-A1- 2 465 508
- EP-A1- 2 510 932
- WO-A1-2009/151315
- WO-A1-2013/083140
- WO-A1-2013/172714
- WO-A2-2014/070016
- US-A1- 2007 098 762
- US-A1- 2009 004 164
- US-A1- 2011 177 044
- US-A1- 2013 143 799
- MONDOULET L ET AL: "Dose-effect of epicutaneous immunotherapy (EPIT) for peanut allergy", ALLERGY, WILEY-BLACKWELL PUBLISHING LTD, UNITED KINGDOM, vol. 64, no. suppl. 90, 10 June 2009 (2009-06-10), page 83, XP008128428, ISSN: 0105-4538 [retrieved on 2010-10-28]

## Description

### FIELD

The present invention relates to a non-digestible oligosaccharide composition for use in preventing or reducing the occurrence of whey protein allergy associated with mycotoxin exposure in infants and toddlers. Also, the invention pertains to infant and/or toddler nutrition, in particular to a non-digestible oligosaccharide composition suitable for use in infant and/or toddler nutrition to prevent or reduce the occurrence of whey protein allergy associated with mycotoxin exposure

### BACKGROUND

Mycotoxins are secondary metabolites produced by moulds and fungi contaminating cereal grains as well as forages, fruits, feed and food products as well as the environment (e.g., soil, water and air through aerosol acquired mycotoxicosis, etc.). Mycotoxins may have dangerous effects on human and animal health. Of particular note are the trichothecene mycotoxins, which are a class of compounds produced by the species *Fusarium graminearum.* This large family of sesquiterpene epoxides are closely related and vary by the position and number of hydroxylations and substitutions of a basic chemical structure. The major trichothecene produced by *Fusarium graminearum* is deoxynivalenol (DON) also known as vomitoxin for its ability to induce vomiting. The impact of DON on nutrient absorption in human intestinal epithelial cells has been investigated in Maresca et al. "The mycotoxin deoxynivalenol affects nutrient absorption in human intestinal epithelial cells" J. Nutr. Vol. 132 (2002) 2723 - 2731, and in Avantaggiato et al. "Evaluation of the intestinal absorption of deoxynivalenol and nivalenol by an in vitro gastrointestinal model, and the binding efficacy of activated carbon and other absorbent materials" Food and Chemical Toxicology vol. 42 (2004) 817 - 824.

Mycotoxins can appear in the food chain as a result of fungal infection of plant products (e.g., forage, grain, plant protein, processed grain by-products, roughage and molasses products), and can either be eaten directly by humans, or introduced by contaminated grains, livestock or other animal feedstuff(s). Since DON frequently occurs in toxicologically relevant concentrations in cereals and grains, it can be qualified as a genuine problem for all humans and animals consuming a diet comprising cereals and/or grains. It is a particular concern for infants, and with that in mind Codex Committees on Contaminants in Food (CCCF) have been dedicated to provide maximum limits for deoxynivalenol levels still deemed acceptable in raw cereal grains such as wheat and barley grain and infant formula. Reference is made to Codex Committee's Agenda for April 2014. (http://ec.europa.eu/food/fs/ifsi/eupositions/cccf/docs/cccf_8_agenda_item_7_en.pdf).

WO 2013/172714 relates to the treatment of all kinds of conditions associated with mycotoxin exposure using a composition comprising a non-digestible oligosaccharide, particularly in fragile infants most vulnerable to the effects of such conditions. Such infants may for instance be prematurely born babies, maturely born babies, infants which are in the adaptation period to solid food, infants and/or toddlers such as with an increased risk for or suffering from allergy, and/or infants and/or toddlers such as with an increased risk for infections, such as infants and/or toddlers attending day care centres, or suffering from infections. Allergies are not mentioned amongst the targeted conditions in WO 2013/172714.

It is also known that infants and young children are often more susceptible than adults to various impairments, including allergies and in particularly food allergies. Such impairments can be acquired during the first days or months of life or they can be present from birth. Genetic heritage plays a significant role, either by direct transmission of a genetic deficiency or by the transmission of a predisposition or susceptibility to such impairments. Alternatively or additionally, environmental (such as allergen exposure, infections, antibiotics) and intrauterine (such as maternal smoking, substance abuse) influences play a significant role, especially early in life, by enhancing the genetic susceptibility of the infant or by directly provoking such impairments. These impairments can be transitory and evolve towards normal status, or they can have a prolonged impact on the subjects. Of particular interest in the context of this invention are the impairments of the gastrointestinal tract and allergies, especially food-mediated allergies in infants.

US 2009/0004164 relates to nutritional compositions comprising non-digestible oligosaccharides and a probioticum suitable for feeding bottle-fed infants in order to restore intestinal flora at birth, thus simulating the microflora of a human milk-fed infant. During early development, the intestinal flora is still immature and its equilibrium is fragile, and it is found that this may have consequences of an increased risk of afflictions such as gastrointestinal infections and atopic diseases such as allergies later in life. Compared to breastfed infants, Lactobacilli in the intestines of bottle-fed infants should be promoted.

EP 2510932 provides a probiotic, *Lactobacillus paracasei* NCC 2461 for use by administration to expecting females and/or lactating mothers, and to their progeny for the reduction or prevention of the development of allergic immune responses in progeny.

US 2013/143799 aims at inducing oral immunological tolerance to the native proteins in the diet, in particular to milk proteins. It is based on the observation that the capacity of a partial milk protein hydrolysate to induce oral immune tolerance was synergistically and significantly increased when concomitantly non-digestible oligosaccharides were also administered via the diet.

EP 2465508 discloses a composition such as a starter infant formula comprising at least one N-acetyl lactosamine, at least one sialylated oligosaccharide and at least one fucosyiated oligosaccharide, and a hydrolysate comprising partialiy and/or extensively hydrolysed proteins, for use in the prevention and/or treatment of skin conditions and skin diseases, in particular atopic dermatitis. Food allergies are generally mentioned.

US 2011/177044 relates to a synbiotic composition comprising the probiotic *Lactobacillus rhamnosus* HN001 and a carbohydrate-based prebiotic such as fiuctooligosaccharides, galactooligosaccharides, human milk oligosaccharides, or combinations thereof, and the use of the composition for the prevention and/or treatment of allergic disease including food allergies.

WO 2013/083140 A1 relates to a nutritional, preferably enteral, composition for use in the treatment of cow's milk allergy in infants allergic to cow's milk, in particular whey protein, containing specific betalactoglobulin peptides, which are able to reduce, in particular abolish the acute symptoms of cow's milk protein allergy.

Mondoulet et al (2009 Blackwell Publishing Lid Allergy 64 (Suppl. 90); 1-98, "Dose-effect of epicutaneous immunotherapy (EPIT) for peanut allergy") report a study comparing the efficacy of different doses of whole peanut protein extracts (PPE) on VIASKIN® in a mice model, wherein the mice were sensitized to peanut using the VIASKIN® device (DBV Technologies, Paris).

WO 2009/151315 A1 discloses methods for feeding of infants delivered via caesarean section and to compositions to be administered to infants delivered via caesarean section; the composition comprising a.) a milk-derived product, said milk-derived product being a milk substrate that is fermented by lactic acid producing bacteria and said milk substrate comprising at least one selected from the group consisting of milk, whey, whey protein, whey protein hydrolysate, casein, casein hydrolysate and/or lactose, b.) less than 10³ cfu lactic acid producing bacteria per g dry weight of the composition, and c.) at least two non-digestible oligosaccharide selected from the group consisting of fructo-oligosaccharides, galacto-oligosaccharides, gluco-oligosaccharides, arabino-oligosaccharides, mannan-oligosaccharides, xylo-oligosaccharides, fuco-oligosaccharides, arabinogalacto-oligosaccharides, glucomanno-oligosaccharides, galactomanno- oligosaccharides, sialic acid comprising oligosaccharides and uronic acid oligosaccharides.

While food allergies are a general concern, none of these documents hint at an increased risk of developing whey protein allergy as a consequence of increased risk or likelihood of intestinal mycotoxin exposure.

The above shows there is a need in the art to address DON-associated health issues, and that there is also a need in the art to address food allergies particularly in the context of young children. Particularly whey protein allergies are a concern early in life.

### SUMMARY OF THE INVENTION

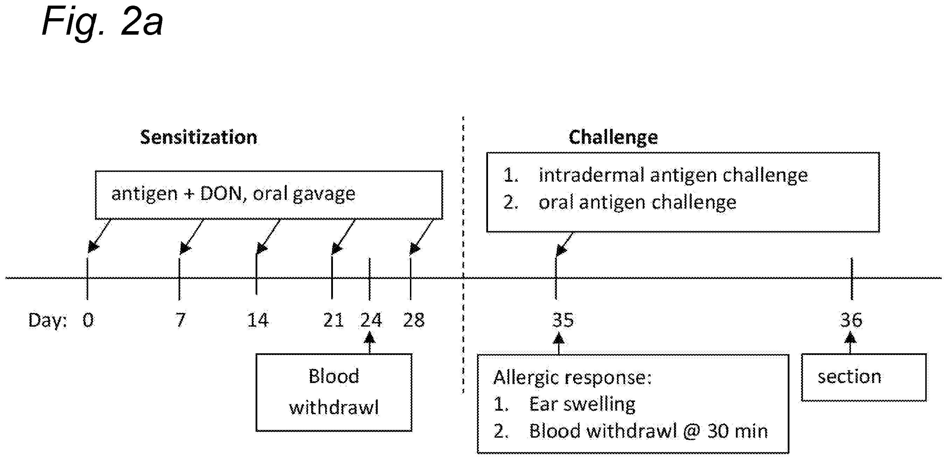

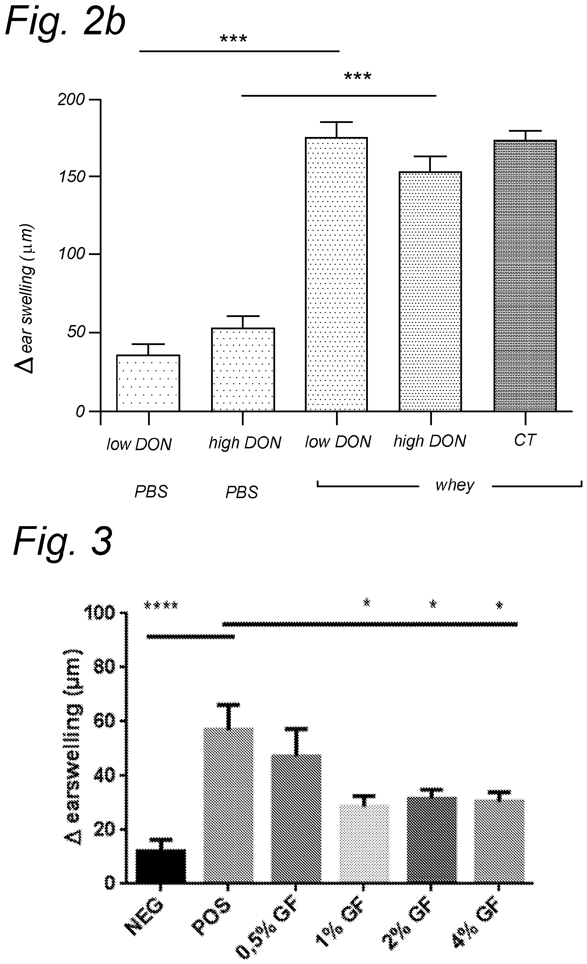

The invention is defined by the claims. The inventors found a correlation between food allergies, particularly whey protein allergies, and exposure to mycotoxins, particularly trichothecene mycotoxins, most particularly deoxynivalenol (DON). It was found that a whey protein allergy (response) is more likely to develop when the infant suffers from exposure to mycotoxins. The increased risk was observed with and without food allergen exposure, albeit that the increased risk induced by DON was more pronounced in the presence of the allergen, i.e the whey protein. An increased risk of developing food allergic response was found most profound in terms of in increased occurrence of a whey protein allergy response in the presence of whey protein. The results of these findings with regard to whey protein are presented in Figure 2.

In one aspect, the invention also pertains to related allergies such as skin allergy and atopic dermatitis.

The inventors' knowledge that mycotoxins, particularly DON, increase food allergy likelihood, particularly whey protein allergy likelihood, the fact that such mycotoxins and particularly DON are frequently present in toxicologically relevant concentrations in cereals and grains, and the fact that infants who consume cereals are more vulnerable to food allergies altogether makes these findings very relevant for infants consuming cereals and grains, i.e. having an increased risk of exposure to mycotoxins such as DON. Those infants will have an increased risk of developing food allergy response(preferably whey protein allergy response) associated with the mycotoxins (or, worded differently: those infants will have an increased risk of developing whey protein allergy response that has resulted from exposure to mycotoxins). The inventors thus recognized there is a need to reduce the detrimental effects of mycotoxin exposure on developing food allergy(preferably whey protein allergy).

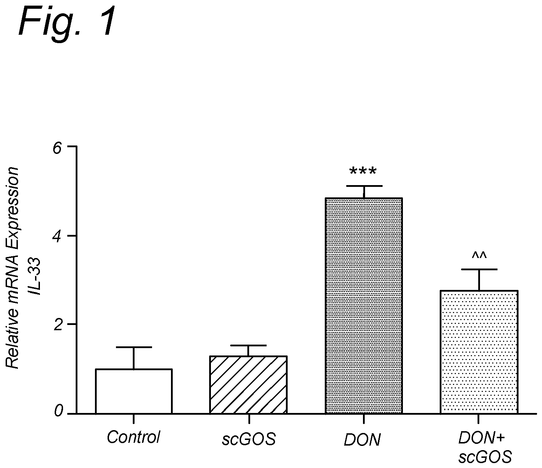

The present inventors also found that non-digestible oligosaccharides, particularly short-chain oligosaccharides, more preferably galactooligosaccharides, can prevent (i.e. reduce the risk of occurrence of) food allergy (preferably whey protein allergy) associated with mycotoxins, preferably DON, all or not in the presence of the food allergens, although preferably in the event of food allergen exposure. More specifically, while mycotoxins result in increased IL-33 mRNA expression, it was found that short-chain oligosaccharides yielded reduced IL-33 mRNA expression. Reference is made to Figure 1. It is known that IL-33 mRNA expression creates a Th2-promoting environment that can lead to the onset of allergy (see for instance Ohno et al. "Interleukin-33 in allergy" Allergy 67 (2012) 1203-1214), and from these results it can be derived that a DON-induced increase in IL-33 production can be significantly reduced by administering said oligosaccharides. The effects of GOS on DON-induced IL-33 mRNA expression and the effects on food allergy (whey protein allergy) associated therewith are unprecedented. These findings were confirmed in Figure 3, directly focussing on the effects of these oligosaccharides on ear swelling in a whey protein allergy model.

Accordingly, in a first aspect the invention pertains to the use of a non-digestible oligosaccharide in the manufacture of a composition for treating an infant suffering from an increased risk of whey protein allergy associated with mycotoxin exposure; and/or reducing the risk of occurrence of or preventing whey protein allergy associated with mycotoxin exposure in an infant suffering from an increased risk of whey protein allergy associated with mycotoxin exposure. The invention also pertains to non-digestible oligosaccharide for use in treating an infant suffering from an increased risk of whey protein allergy associated with mycotoxin exposure; and/or use in reducing the risk of occurrence of or preventing whey protein allergy associated with mycotoxin exposure in an infant suffering from an increased risk of whey protein allergy associated with mycotoxin exposure. Also, the invention pertains to a non-therapeutic method for providing nutrition to an infant suffering from an increased risk of whey protein allergy associated with mycotoxin exposure, comprising administering non-digestible oligosaccharide to said infant. The food allergy is preferably whey protein allergy associated with mycotoxin exposure. The infant consumes cereals or cereal-comprising products on a daily basis. There is an increased risk that such cereals or cereal-comprising products are contaminated with mycotoxins, and these infants thus suffer from an increased risk of developing whey whey protein allergy associated with mycotoxin exposure, more than those infants not (yet) consuming cereals or cereal-comprising products, or at lesser scales.

The composition comprising the non-digestible oligosaccharides according to the invention is free of toxins, it addresses the issues of mycotoxin contamination of other cereal-containing products in the infant/toddler's diet.

In the above context, the term 'infants' encompasses toddlers, and particularly infants of at least 6 months of age and toddlers of 1 - 6 years, preferably 12 - 36 months of age could benefit from these insights. The infants and toddlers frequently (daily) consume cereal-based foodstuffs with an increased likelihood of intestinal exposure to mycotoxins such as DON. It is a preferred target group as milk and whey proteins form an important part of their diet. The infants and toddlers preferably consume whey protein on a frequent (daily) basis. The associated risk of developing whey protein allergen (response) associated with increased exposure to DON and the food allergen is significantly reduced by administering non-digestible oligosaccharides, preferably galacto-oligosaccharides, all or not in combination with fructo-oligosaccharides and/or fructopolyaccharides according to the invention. The invention is particularly directed to the above-defined groups of toddlers who consume relatively large amounts of cereals including mycotoxin-contaminated cereals and cereal products, thus having a high risk of getting exposed to mycotoxins and are vulnerable to mycotoxin-associated whey protein allergies.

In the art it is believed that DON could also be a risk to infants early in life because it is suggested that breastfeeding women who are exposed to DON could transfer it onto the infant through breastmilk. Also, it is believed that the same can occurr when pregnant women are exposed to DON. In one aspect, infants at increased risk of being exposed to DON encompass newborn infants. In one aspect, the invention thus pertains to the use of a non-digestible oligosaccharide in the manufacture of a composition for administration to pregnant women in at least the third trimester of pregnancy, and, after delivery, to the breastfeeding women who are at increased risk of DON-exposure, in order to reduce the risk of occurrence of or preventing whey protein allergy (response) associated with mycotoxin exposure in the baby in infancy. The invention also pertains to non-digestible oligosaccharide for use in administration to pregnant women in at least the third trimester of pregnancy, and, after delivery, to the breastfeeding women who are at increased risk of DON-exposure, in order to reduce the risk of occurrence of or preventing whey protein allergy (response) associated with mycotoxin exposure in the baby in infancy. Also, the invention pertains to a method for administering a (nutritional) composition to pregnant women in at least the third trimester of pregnancy, and, after delivery, to the breastfeeding women who are at increased risk of DON-exposure, in order to reduce the risk of occurrence of or preventing whey protein allergy (response) associated with mycotoxin exposure in the baby in infancy. The associated risk of developing whey protein allergy (response) associated with mycotoxin exposure is associated with the mother's increased exposure to DON, and the food allergen development is significantly reduced by administering non-digestible oligosaccharides, preferably galacto-oligosaccharides, according to the invention. This embodiment preferably concerns infants up to 1 years of age, more preferably 0 - 6 months of age.

### LIST OF FIGURES

Figure 1. ScGOS selectively counteracted induction of IL-33 mRNA expression levels as well as its localization in distal small intestine of DON-treated mice. The mice were fed a control diet or a diet supplemented with scGOS for 2 weeks before an oral DON gavage. After 6h the IL-33 mRNA levels were measured by qRT-PCR (A). n = 5-6 animals per group. Results are relative mRNA expression as mean ± SEM. (***P < 0.001; significantly different from the control group. ^^P < 0.01; significantly different from the DON-treated animals). For the immunohistochemical staining, Swiss-rolled paraffin sections obtained from distal small intestine were detected by antibodies for IL-33 (B,C,D) N= 5-6 mice/group (2-3 sections/animal). Magnification 400x.
Figure 2a. Allergy *in vivo* mouse model outline; and
Figure 2b. The effect of DON on ear swelling as an allergy response indicator is presented without (PBS) and with whey protein present.
Figure 3. The effect of various galacto-oligosaccharide concentrations on ear swelling as a result of trichothecene mycotoxin exposure associated whey protein allergy. The galacto-oligosaccharides form the predominant part of a mixture of galacto- and fructooligosaccharides ('GF') added to the diet.

### LIST OF EMBODIMENTS ALSO DESCRIBED BUT NOT NECESSARILY PART OF THE INVENTION AS CLAIMED

1. Use of a non-digestible oligosaccharide in the manufacture of a composition for providing nutrition to an infant suffering from an increased risk of food allergy; and/or reducing the risk of occurrence of or preventing food allergy in an infant suffering from an increased risk of food allergy.
2. Use according to embodiment 1, wherein said infant suffers from an increased risk of peanut allergy, chicken protein allergy or whey protein allergy, preferably at least whey protein allergy.
3. Use according to any of the preceding embodiments, wherein said infant is at increased risk of trichothecene mycotoxin exposure, and wherein said infant is preferably at increased risk of food allergen exposure.
4. Use according to any of the preceding embodiments, wherein said infant consumes cereal-comprising products daily.
5. Use according to any of the preceding embodiments, wherein said infant suffers from an increased risk of trichothecene mycotoxin exposure associated food allergy, preferably trichothecene mycotoxin exposure associated whey protein allergy, more preferably deoxynivalenol exposure associated whey protein allergy.
6. Use according to any of the preceding embodiments, wherein the non-digestible oligosaccharide has a degree of polymerisation (DP) of 2 - 10.
7. Use according to embodiment 6, wherein the non-digestible oligosaccharide comprises a galactooligosaccharide and/or a fructooligosaccharide, preferably at least a galactooligosaccharide, more preferably at least transgalactooligosaccharide.
8. Use according to any of the preceding embodiments, wherein the non-digestible oligosaccharide comprises a short-chain galactooligosaccharide and/or a short-chain fructooligosaccharide.
9. Use according to any of the preceding embodiments, wherein said infant is a baby or weaning infant or weaning toddler, preferably a toddler between 12 to 36 months old.
10. A method for providing nutrition to or treating an infant suffering from an increased risk of food allergy, and/or reducing the risk of occurrence of or preventing food allergy in an infant suffering from an increased risk of food allergy, comprising administering non-digestible oligosaccharides to said infant.
11. The method according to embodiment 10, wherein said infant follows a high-cereal diet comprising daily consumption of cereal-comprising products.
12. The method according to embodiment 10 or 11, wherein said infant suffers from an increased risk of trichothecene mycotoxin exposure associated food allergy, preferably trichothecene mycotoxin exposure associated whey protein allergy.

### DETAILED DESCRIPTION OF THE INVENTION

The invention is defined by the claims. The composition according to the invention comprises a non-digestible oligosaccharide and may comprise a water-soluble non-digestible oligosaccharide composition, such as a galacto-oligosaccharide and/or a fructo-oligosaccharide, preferably at least a galacto-oligosaccharide. In general, the compositions and methods described here make use of a non-digestible oligosaccharide composition, which is described in detail below. Such a composition may comprise a non-digestible oligosaccharide such as a galacto-oligosaccharide or a fructo-oligosaccharide, preferably at least a galacto-oligosaccharide. Throughout the description, with "non-digestible oligosaccharide composition" it is understood a composition comprising a non-digestible oligosaccharide, more preferably a nutritional composition. In one embodiment, the nutritional composition may be given to a pregnant woman in at least the third trimester of pregnancy, or, after delivery, to the breastfeeding woman or the newborn baby, in order to prophylactically treat the newborn infant, preferably up to 1 years of age. In a preferred embodiment, the nutritional composition is administered to an infant of at least 6 montsh of age, or toddler preferably 1 - 6 years of age. The toddlers are particularly targeted with the compositions of the invention.

Trichothecene mycotoxins such as deoxynivalenol are known to cause a number of severe effects on individuals unfortunate to ingest them or to be exposed to them. The inventors found that increased risk of occurrence of food allergy is amongst those, and that the risk for food allergy associated with exposure to a trichothecene mycotoxin such as deoxynivalenol could be reduced by use of a non-digestible oligosaccharide composition such as an scGOS composition ('sc' meaning 'short-chain') or scFOS composition, preferably a scGOS composition associated with exposure to a trichothecene mycotoxin such as deoxynivalenol. The effects have been demonstrated for whey protein allergy as found attached.

For example, a subject may be defined as being "treated" if whey protein allergy associated with mycotoxin exposure is significantly inhibited (i.e., by 50% or more) relative to controls. The inhibition may be by at least 75% relative to controls. By the term "treatment" we mean to also include prophylaxis and prevention (including reducing the risk of occurrence) of any of the conditions or symptoms specified and/or the severity of any of the conditions or symptoms specified. In the context of the invention, the terminology 'food allergy' and 'food allergy response' are used interchangeably. The same holds for 'whey protein allergy' and 'whey protein allergy response' .

In the (prophylactic) treatment according to the invention, the non-digestible oligosaccharide is administered in an amount effective for treatment, i.e. intended to mean that amount of a substance that will elicit the biological or medical allergic response of the subject that is being sought by a researcher, veterinarian, medical doctor or other clinician. The term also encompasses the amount of the non-digestible oligosaccharide that will prevent or reduce the risk of occurrence of whey protein allergy associated with mycotoxin exposure that is sought to be prevented by a researcher, veterinarian, medical doctor or other clinician.

Where the term "(trichothecene) mycotoxin exposure associated whey protein allergy" is used in this document, this should be considered as referring to a whey protein allergy caused or resulted by or associated with exposure of an individual, organ, tissue or cell (as the case may be) to a trichothecene mycotoxin. In the context of the invention, the exposure associated whey protein allergy is preferably intestinal exposure associated whey protein allergy. The wording 'whey protein allergy (response) associated with (trichothecene) mycotoxin exposure' and 'whey protein allergy (response) that resulted from (trichothecene) mycotoxin exposure' are used interchangeably, realizing however that mycotoxin exposure does not result in a direct whey protein allergy response, but makes the subject more vulnerable to develop such whey protein allergy response. Without wishing to be tied down to any theory, the inventors believe that the observed increase in whey protein allergy response is associated with changes to the intestinal barrier induced by mycotoxins and particularly DON, thus disadvantageously making it easier for whey protein allergens to pass. The inventors also believe that the effects for whey protein allergy (response) stand model for other allergies (food allergy, skin allergy, atopic dermatitis) associated with mycotoxin exposure, particularly in infants and toddlers who consume myxoctoxin-contaminated cereals and cereal products.

### Non-digestible oligosaccharides

Based on dry matter, the non-digestible oligosaccharide composition comprises, if any amount, less 0.2 mg/kg, more preferably less than 0.1 mg/kg, even more preferably less than 50 mcg/kg mycotoxins, but more preferably mycotoxin levels are below detection limits (less than 5 mcg/kg mycotoxins). Analytical methods for assessing mycotoxin presence in foodstuffs are available and discussed further on. The invention addresses the issues associated with exposure to mycotoxins which may be present in cereals such as grains and wheat in an infant's diet, but it is not part of the invention to contribute to mycotoxin exposure at all.

In one embodiment, the non-digestible oligosaccharide composition preferably comprises a neutral non-digestible oligosaccharide.

The non-digestible oligosaccharide may be water-soluble, as determined according to the method disclosed in L. Prosky et al, J. Assoc. Anal. Chem 71 : 1017-1023, 1988.

The non-digestible oligosaccharide may comprise an oligosaccharide with a degree of polymerisation (DP) of 2 to 200. The non-digestible oligosaccharide is preferably an oligosaccharide having a degree of polymerisation (DP) of 2 to 60, more preferably 2 - 40, even more preferably 2- 20, most preferably 2 - 10, particularly 2 - 8, 2 - 7, 2 - 6, 2 - 5. The average DP of the non-digestible oligosaccharide may be below 100, such as below 50, such as below 20, such as below 10. The non-digestible oligosaccharide may have an average DP of 2-10, such as 2-8, 2-7, 2-5, and any other range within these parameters. In a particularly preferred embodiment, the non-digestible oligosaccharide is a galactooligosaccharide or a fructooligosaccharide, or a mixture thereof, having a DP of 2 - 10, such as 2-8, 2-7, 2-6, 2 - 5; and/or having an average DP 2 - 10, such as 2-8, 2-7, 2-6, 2-5. In the context of the invention, these oligosaccharides having a DP 2-10 may be addressed as short chain GOS ('scGOS') and short chain FOS ('scFOS'), respectively. In one embodiment, a scGOS is particularly preferred.

As used in this document, the term "degree of polymerisation" or "DP" is intended to refer to the total number of saccharide units in an oligo- or polysaccharide chain. The "average DP" is intended to refer to the average DP of oligosaccharides or polysaccharide chains in a composition, without taking monosaccharides and digestible disaccharides such as lactose into account (which may be removed if present).

The non-digestible oligosaccharide may be one that is not digested in the intestine by the action of digestive enzymes present in the human upper digestive tract (small intestine and stomach). The non-digestible oligosaccharide may be fermented by the human intestinal microbiota. For example, glucose, fructose, galactose, sucrose, lactose, maltose and the maltodextrins are considered digestible. The oligosaccharide raw materials may comprise monosaccharides such as glucose, fructose, fucose, galactose, rhamnose, xylose, glucuronic acid, GalNac etc., but these are not part of the non-digestible oligosaccharides as used in the present description.

The composition comprising a non-digestible oligosaccharide (and the associated methods of using the composition as described herein) may include a mixture of non-digestible oligosaccharides. The non-digestible oligosaccharide may be selected from the group consisting of galacto-oligosaccharide, such as transgalacto-oligosaccharide, xylooligosaccharide, arabino-oligosaccharide, arabinogalacto-oligosaccharide, glucooligosaccharide, such as gentio-oligosaccharide and cyclodextrin, glucomanno-oligosaccharide, galactomanno-oligosaccharide, mannan-oligosaccharide, chito-oligosaccharide, fructo-oligosaccharide, such as inulin, non-digestible dextrin, uronic acid oligosaccharide, sialyloligosaccharide, such as 3-SL, 6-SL, LSTa.b.c, DSLNT, S-LNH, DSLNH, and fuco-oligosaccharide, such as (un)sulphated fucoidan OS, 2-FL, 3-FL, LNFP I, II, III, V, LNnFPI, LNDH, and mixtures thereof. The non-digestible oligosaccharide may particularly be selected from the group consisting of galacto-oligosaccharide, such as transgalacto-oligosaccharide, and fructo-oligosaccharide, particularly scGOS and/or scFOS. In a most preferred embodiment, the non-digestible oligosaccharide comprises at least galacto-oligosaccharide, particularly scGOS.

Although the composition may comprise only a single non-digestible oligosaccharide, we also describe compositions with two different non-digestible oligosaccharides, i.e. non-digestible oligosaccharide A and non-digestible oligosaccharide B. Non-digestible oligosaccharide A and non-digestible oligosaccharide B may have a different type of glycosidic linkage, a different degree of polymerisation and/or a different monosaccharide composition.

The non-digestible oligosaccharide may comprise a particular glycosidic linkage diversity. The term "glycosidic linkage" may be used in this document to refer to a C-O-C linkage formed between the rings of two cyclic monosaccharides by the elimination of water. Glycosidic linkages differ in that they covalently bind carbon atoms in the monosaccharide units at differently numbered positions, and/or that they form α- (alpha) or β- (beta) bonds. Examples of different glycosidic linkages occurring in non-digestible saccharides are β(1 ,3), α(1 ,4), β(2,1), α(1 ,2), and β(1 ,4) linkages. The glycosidic linkages in the non-digestible oligosaccharide may comprise at least 40% β(1 ,4) and/or β(1 ,6) glycosidic linkages, such as at least 75%.

For example, at least 60%, such as at least 75% such as 90%, such as 98% of the total monosaccharide units of the non-digestible oligosaccharide, may comprise monosaccharides selected from the group consisting of galactose (gal), fructose (fru) and glucose (glu) monosaccharides.

The non-digestible oligosaccharide may comprise an oligosaccharide selected from the group consisting of β-galacto-oligosaccharide, α-galacto-oligosaccharide, and galactan. β-galacto-oligosaccharide is also sometimes referred to as transgalacto-oligosaccharide. For example, the non-digestible oligosaccharide may comprise galacto-oligosaccharides with β(1,4), β(1,3) and/or β(1 ,6) glycosidic bonds and a terminal glucose. Transgalactooligosaccharide is for example available under the trade name Vivinal®GOS (Borculo Domo Ingredients, Zwolle, Netherlands), Bi2muno (Clasado), Cup-oligo (Nissin Sugar) and Oligomate55 (Yakult). Fructooligosaccharides may be inulin hydrolyzate products, preferably with a DP and/or average DP within the aforementioned (sub-)ranges; such FOS products are for instance commercially available as Raftilose P95 (Orafti) or with Cosucra.

For example, a transgalacto-oligosaccharide with an average DP below 10, such as 6 may be used as the non-digestible oligosaccharide.

The galacto-oligosaccharide (GOS) may comprise a short chain galactooligosaccharide (scGOS).

The non-digestible oligosaccharide may be present in the composition at any suitable concentration, preferably in a therapeutically effective amount or "amount effective for treating" as defined above. For example, where the composition comprises a liquid, such as made-up formula milk or a made-up growing up milk, the non-digestible oligosaccharide may be present at for example, between 0.01 g/100 ml to 10 g/100 ml (0.1 to 100 g/l), 0.05 g/100 ml to 5.0 g/100 ml, 0.10 g/100 ml to 2.0 g/100 ml, 0.20 g/100 ml to 1.0 g/100 ml or 0.10 g/100 ml to 1.0 g/100 ml, etc.. In one embodiment, the level of non-digestible oligosaccharides, preferably comprising galactooligosaccharide(s) and optionally fructo-oligosaccharide(s), is between 0.10 - 5.0 g/100 ml.

Compositions containing fructo-oligosaccharide(s) and/or galacto-oligosaccharide(s) are described in detail in WO 2000/08948 (and English language equivalents such as AU 766924), WO 2005/039597 and WO 2005/110121.

In one embodiment, the composition comprises a further non-digestible oligosaccharide with an average DP of 20 - 100, preferably 20 - 50, a suitable example being fructopolysaccharide (FPS) such as Raftilline (Orafti). In a preferred embodiment, the composition comprises an oligosaccharide mixture comprising short-chain galacto-oligosaccharides and at least one of short-chain fructooligosaccharides or long-chain fructopolysaccharides. In one embodiment, the composition comprises scGOS and FPS in a weight ratio of 4:1 - 19:1. The level of non-digestible oligosaccharides is preferably between 0.10 - 5.0 g/100 ml.

### Trichothecene mycotoxins

The '12,13-epoxytrichothecenes' (also known as "trichothecene mycotoxins" and "trichothecenes") are a large group of chemicals characterised by a double bond between C9 and C10 and an epoxy ring at the C12- C13 position in the chemical structure. The trichothecene mycotoxins are a group of related and biologically active mycotoxins often wrongly referred to as the *Fusarium* toxins as several other fungal genera including Trichoderma, *Stachybotrys, Verticimonosporium, Cephalosporium* and *Myrothecium* can also produce them. Although the number of compounds of this type runs into the hundreds, only a few have been shown to be agriculturally important. However the fusaria are by far the most important mycotoxin-producing species occurring widely in field crops with more than 20 species of *Fusarium,* including *F. poae, F. sporotrichioides, F. moniliforme, F. culmorum,* and F. *graminearum* among the most important trichothecene producers.

Trichothecene mycotoxins are often classified as Group A and Group B compounds (also known as Type A and Type B) depending on whether they have a side chain on the C7 atom. The most commonly reported Group A trichothecenes include, T-2 toxin, HT-2 toxin, neosolaniol, monoacetoxy scirpenol and diacetoxyscirpenol. Common group B trichothecenes are deoxynivalenol, nivalenol, 3- and 15- acetoxynivalenol and fusarenon X (a separate fact sheet is devoted to deoxynivalenol). In addition to producing mycotoxins these fungi include important plant pathogens that cause a number of serious diseases in growing crops. Deoxynivalenol is a "Type B" or "Group B" trichothecene mycotoxin (in Formula II), R₁ = OH, R₂ = H, R₃ = OH, R₄ = OH in deoxynivalenol). Another group of trichothecenes which are generally more acutely toxic than T-2 toxin are known as the macrocyclic trichothecenes produced by mould species such as *Stachybotrys atra.* These include the satratoxins, verrucarins and roridins. All of these mycotoxins are encompassed in the context of the invention, albeit that deoxynivalenol is the most profound mycotoxin addressed by the invention. In one embodiment, DON includes DON derivatives such as acetylated DON.

### Deoxynivalenol (DON)

The trichothecene mycotoxin targeted may preferably comprise deoxynivalenol or DON derivatives. Deoxynivalenol is nearly always formed before harvest when crops are invaded by certain species of *Fusarium* such as *F. graminearum* and *F. culmorum.* These two species are important plant pathogens and cause *Fusarium* heat blight in wheat and *Gibberella* ear rot in maize. Deoxynivalenol is thermally stable so once formed it is likely to persist through storage and the food chain.

Deoxynivalenol is also known as DON, vomitoxin, dehydronivalenol or 12,13-epoxy-3,7,15-trichothec-9-en-8-one. It has the molecular formula C₁₅H₂₀O₆. The IUPAC name is (3α, 7α)- 3, 7, 15- trihydroxy- 12, 13- epoxytrichothec- 9- en- 8- one. Deoxynivalenol has CAS number 51481-10-8, PubChem number 40024, ChemSpider number 36584, KEGG number C09747 and ChEMBL number CHEMBL513300. Deoxynivalenol is one of the more polar trichothecenes with a molecular weight of 296.32. It contains one primary and two secondary hydroxyl groups and is soluble in water and polar solvents such as methanol and acetonitrile. Unlike many of the other trichothecenes the deoxynivalenol molecule contains a conjugated carbonyl system and this results in some UV absorbance that assists its detection by TLC or HPLC methods.

At the cellular level, the primary toxic effect of DON is inhibition of protein synthesis by binding to the 60S ribosomal subunit, which interferes with peptidyltransferase (Betina, Chem. Biol. Interact. 71: 105-146 (1989); Weber et al., Biochem. 31: 9350-9354 (1992)). DON can cause anorexia and emesis in animals (Scott et al. Proc. natl. Acad. Sci. USA 89: 5398-5402 (1992)). Other toxic effects of DON include skin irritation, hemorrhaging, hematological changes, human lymphocyte blastogenesis impairment, radiomimetic effects, apoptosis and immunotoxicity (Scott *et al.* ibid.). The disadvantageous whey protein allergy (response)-stimulating effects of DON have not yet been acknowledged in the art.

DON is primarily found as a contaminant in grains that are infected with the above fungi. It has also been implicated as a chemical warfare agent. Currently, the only means for eliminating DON from human and animal foodstuffs is to detect DON in food and to remove any contaminated foodstuffs from the food supply. In contaminated cereals 3- and 15-acetyl deoxynivalenol can co-occur in significant amounts with deoxynivalenol. It is chemically very stable. It underscores the need for products that could balance the effects of (intestinal) DON exposure, as such exposure is - sadly - difficult to avoid.

### Cereal-diet; products affected and Natural Occurrence

Surveys have shown that trichothecenes and particularly deoxynivalenol is a frequent contaminant of grains such as wheat, buckwheat, barley, oats, triticale, rye, maize, sorghum and rice, all encompassed in the term 'cereals'. Past surveillance of cereals commonly targeted deoxynivalenol only although other trichothecenes are highly likely to be present and the recent trend is to screen for the range of related compounds that may be expected to occur. EFSA reports that the highest deoxynivalenol levels are observed in wheat, maize and oat grains and derived products. Concentrations have been reported up to as high as 9 mg/kg in barley and 6 mg/kg in wheat. Because it is a stable compound it has also been detected in a range of processed cereal products including breakfast cereals, bread, noodles, infant foods, malt and beer.

Since the invention particularly addresses the issues of whey protein allergies associated with mycotoxin exposure in infants, particularly weaning infants and toddlers, it is preferred to target those infants who are given a high-cereal diet, i.e. eating cereals or cereal-comprising foodstuffs on a frequent or even daily basis. Participating in such a dietary regimen, there is increased likelihood of trichothecene exposure, and consequently and - as shown by the inventors - an increased risk of developing whey protein allergy associated with mycotoxin exposure. The infants are preferably also exposed to the food allergen whey protein frequently, preferably on a daily basis.

The targeted infant or toddler preferably consumes a high cereal diet with wheat, maize and oat grains and derived products (i.e. cereal-comprising products), including bread, rice and pasta. A 'high cereal diet' preferably means a dietary regimen involving consumption of cereal-comprising products comprising bread, rice and pasta, more preferably at least wheat, maize and/or rice, on a daily basis. In one embodiment, the cereal-comprising product may comprise a component selected from the group consisting of whole cereal, cereal flour, milled cereal, ground cereals, cereal starch, and cereal fibre. The cereal-comprising product may for example comprise a component selected from the group consisting of cereal flour, ground cereal and milled cereal. Risk for DON exposure is most profound when consuming products comprising wheat, maize or rice. The invention is particularly directed to the above-defined groups of toddlers who consume mycotoxin-contaminated cereals and cereal products, or are at increased risk thereof.

The analytical method of choice for quantitatively detecting mycotoxins in foods today is often GC either with electron capture or mass spectrometric detection (MS). Recently, LC-MS, has been employed for the determination and identification of trichothecenes at trace levels. In addition, reliable and quite sensitive HPLC methods have been developed for some of the Group B compounds. Methods with sensitivities of about 5 µg/kg are available.

When assessing a foodstuff for mycotoxin contamination, representative sampling is important. Mycotoxin distribution in bulk grain has been poorly studied and sampling plans derived for other mycotoxins such as aflatoxin and ochratoxin A should be followed.

### Composition

The composition comprising non-digestible oligosaccharide(s) according to the invention may comprise an enteral composition, i.e., anything that is enterally administered, such as orally. In particular, the enteral composition may comprise a foodstuff, such as nutritional composition or nutritional food. As used in this document, the term "enteral" is intended to refer to the delivery directly into the gastrointestinal tract of a subject (e.g. orally or via a tube, catheter or stoma).

After delivery, the composition may be administered either to the infant via the breastfeeding mother or may be administered directly to the infant, the latter being preferred. If the composition is given to the mother, it is preferably a supplement.

In view of the above, the composition preferably comprises an infant and/or toddler nutrition, such as an infant and/or toddler formula. The composition may comprise a children's nutritional product. It may comprise a pediatric nutritional product or formula, a toddler nutritional formula, growing up milk, human milk supplement or medicinal food.

The composition may in one embodiment be used as an infant formula. The composition may be applied as a complete nutrition for infants. Such food may comprise lipid, protein and carbohydrate and may be administered in liquid form. The infant formula is preferably a 'growing up' formula for infants of at least 6 months of age.

In a further embodiment, the composition may comprise a ready-to-use liquid food, e.g. is in a ready-to-feed liquid form. A packed ready-to-use liquid food may involve fewer steps for preparation than a powder to be reconstituted and hence may involve a reduced chance of contamination by harmful micro-organisms.

The composition described here may comprise an infant and/or toddler nutrition which for example comprises between 5 and 50 en% lipid, between 5 and 50 en% protein, between 15 and 90 en% carbohydrate and non-digestible oligosaccharide A and/or B. In some embodiments, the composition may comprise an infant and/or toddler nutrition comprising between 35 and 50 en% lipid, between 7.5 and 12.5 en% protein and between 35 and 80 en% carbohydrate (en% is short for energy percentage and represents the relative amount each constituent contributes to the total caloric value of the preparation).

The composition comprising non-digestible oligosaccharides may comprise lipids, such as vegetable lipids, and/or at least one oil selected from the group consisting of fish, animal, algae and bacterial oil. The composition may comprise long chain polyunsaturated fatty acids (LC-PUFA), such as eicosapentaenoic acid (EPA) and/or docosahexaenoic acid (DHA), and/or docospentanenoic acid (DPA), and/or arachidonic acid (ARA).

The composition comprising non-digestible oligosaccharides may comprise proteins. The proteins used in the nutritional preparation may be selected from the group consisting of non-human animal proteins (such as milk proteins, including caseins and whey proteins, meat proteins and egg proteins), vegetable proteins (such as soy protein, wheat protein, rice protein, potato protein and pea protein), hydrolysates (partially and/or extensively), free amino acids and mixtures thereof. The protein of the infant nutrition may be selected from the group consisting of hydrolysed milk protein (e.g. hydrolysed casein and/or hydrolysed whey protein), hydrolysed vegetable protein and/or amino acids. The use of these proteins may reduce the allergic reactions of the infant and/or toddler and/or increase protein absorption. The protein source may be extensively and/or partially hydrolysed. The protein source may be extensively hydrolysed whey protein derived from cow's milk.

The composition comprising non-digestible oligosaccharides may comprise digestible carbohydrates. The digestible carbohydrates used in the nutritional preparation may be selected from the group consisting of sucrose, lactose, maltose, galactose, glucose, fructose, corn syrup solids, starch and maltodextrins, and mixtures thereof, such as lactose.

The composition comprising non-digestible oligosaccharides may comprise minerals, trace elements and vitamins, choline, taurine, carnitine, myo-inositol and/or mixtures thereof. The composition comprising non-digestible oligosaccharides may comprise nucleotides. The composition may comprise cytidine 5'-monophospate, uridine 5'-monophospate, adenosine 5'-monophospate, guanosine 5'-monophospate, and inosine 5 '-monophospate.

The composition comprising non-digestible oligosaccharides may comprise a non-fermented composition. Fermentation by micro-organisms results in a lowering of the pH. The composition may have a pH above 5.5, such as 6.0, such as 6.5 in order to reduce damage to teeth. The pH may be between 6 and 8.

The composition may be formulated so that it does not have an excessive caloric density, however still provides sufficient calories to feed the subject. Hence, the liquid food may have a caloric density between 0.1 and 2.5 kcal/ml, such as a caloric density of between 0.4 and 1.2 kcal/ml, such as between 0.55 and 0.75 kcal/ml.

The composition comprising non-digestible oligosaccharides may have a viscosity between 1 and 60 mPa.s, such as between 1 and 20 mPa.s, such as between 1 and 10 mPa.s, such as between 1 and 6 mPa.s. The viscosity of the liquid may be determined using a Physica Rheometer MCR 300 (Physica Messtechnik GmbH, Ostfilden, Germany) at shear rate of 95 s⁻¹ at 20°C. The low viscosity ensures a proper administration of the liquid, e.g. a proper passage through the hole of a nipple. Also this viscosity closely resembles the viscosity of human milk. Furthermore, a low viscosity results in a normal gastric emptying and a better energy intake, which is essential for infants and/or toddlers which need the energy for optimal growth and development. The composition may be prepared by admixing a powdered composition with water. Normally infant formula is prepared in such way.

The composition described here may be made up as a packaged power composition wherein said package is provided with instruction to admix the powder with a suitable amount of liquid, thereby resulting in a liquid composition with a viscosity between 1 and 60 mPa.s, for instance when measured at a shear rate of 95 s⁻¹ at 20°C.

The composition comprising non-digestible oligosaccharides may have a long shelf life. For example, it may be shelf stable at ambient temperature for at least 6 months, such as at least 12 months, where it is in a liquid, ready-to-feed form.

### Growing up milk and cereals

The composition comprising non-digestible oligosaccharides may comprise a cereal or a growing up milk. Cereal components are an important part of the infant's diet, and are usually one of the first non-breast milk and non-infant formulae components introduced into the diet of infants. In one embodiment, the non-digestible oligosaccharide composition may therefore comprise a children's nutritional composition provided as a growing-up milk or cereal. In an embodiment, sources of carbohydrate for use in the growing-up milk or cereal may include maltodextrin, fructose, lactose, prebiotics, resistant starch, starch, and any combinations thereof. It is reiterated that it is preferred that cereals comprised in the non-digestible oligosaccharide composition of the invention are free from mycotoxins, in order to avoid contributing to the problem of increased likelihood of developing whey protein allergy associated with mycotoxin exposure. A cereal component optionally present in the non-digestible oligosaccharide composition according to the invention does not contribute to the above preferred definitions of high cereal diets. The composition such as infant formula or growing up milk of the invention is thus free of toxins.

The cereal component in the non-digestible oligosaccharide composition of the invention may comprise a component selected from the group consisting of whole cereal, cereal flour, milled cereal, ground cereals, cereal starch, and cereal fibre. The cereal component may for example comprise a component selected from the group consisting of cereal flour, ground cereal and milled cereal. The cereal flour may in some embodiments comprise cereal flour which is dextrinised by heat treatment and/or cereal flour which has been enzyme treated in order to degrade the cereal starch.

The non-digestible oligosaccharide composition described here may comprise a precooked cereal component, such as precooked cereal flour. The term "precooked cereal flour" indicates flour obtained by the process whereby flour, in granular and crystalline structure is swelled and transformed, for example in a continuous amorphous phase, in the presence of heat and water, dried (e.g. using drum drying or extrusion cooking) and ground. The precooked flour may comprise between 5 and 15 wt.% protein based on the total dry weight of the precooked flour. The use of precooked flour may be such that the final product has a reduced content of thermo-resistant spores compared to the use of non-precooked flour. Furthermore, the use of precooked may be such that the viscosity of the composition is more stable after reconstitution of the product with a warm liquid. This is in contrast to the situation wherein solely non-precooked flour is used. In the latter case the viscosity gradually increases with time. The precooked flour if present may have a degree of gelatinisation of at least 50%, such as at least 75%. This gives better water holding capacity (WHC), resulting in an improved product (e.g. stability and palatability). The WHC of the precooked material may be between 2 and 10 g water/g dry matter precooked material, such as between 2.5 and 5 g water/g dry matter precooked material. The WHC can be determined as described by Pinnavaia and Pizzirani (Starch/Stärke 50 (1998) nr. 2-3, S. 64-67).

The non-digestible oligosaccharide composition described here may comprise at least one cereal selected from the group consisting of rice, millet, sorghum, wheat, barley, buckwheat, maize (corn), fonio, oats, rye, triticale, teff, wild rice, spelt, amaranth, quinoa and starchy root crops. Starchy root crops may be selected from the group consisting of potato, sweet potato, cassava, yams, aroids, oca, ulluco and mashua.

The composition according to the invention may be gluten free. The intake of gluten by infants below 6 month of age may result in gastro-intestinal damage. In some embodiments, therefore, the composition may comprise one or more cereal components selected from the group consisting of rice, maize and millet, sorghum, teff, oat and starchy root crops. For example, the composition may comprise one or more cereal components selected from the group consisting of rice, maize and millet, teff, and oat. The composition may consist of rice, maize and millet, sorghum, teff, oat, starchy root crops and mixtures thereof. The cereal may be selected from the group consisting of rice, maize, oat, teff and millet. The cereal part of the composition may comprise mixture of cereal components. Typically the cereal is processed as defined in EU directive 96/5/EC.

The composition described here may comprise between 10 and 99 g cereal component per 100 g dry weight of the composition, such as between 20 and 90 g, such as between 25 and 80 g.

Where the composition comprises a growing-up milk or cereal formulated for children between the ages of 1 to 6 years, vitamins and minerals may be added in varying amounts and ranges based on a per-serving basis. In an embodiment, one serving of the growing-up milk or cereal may contain from about 15% to about 50% of the Estimated Average Requirement (EAR) for children between the ages of 1 and 6 years for the following nutrients: vitamin E, vitamin K, niacin, pantothenic acid, vitamin B12, biotin, choline, potassium, magnesium, phosphorus, chloride, copper, selenium, fluoride, and any combinations thereof. In an embodiment, one serving of the growing-up milk or cereal may contain from about 20% to about 30% of the EAR for children between the ages of 1 and 6 years for the following nutrients: vitamin E, vitamin K, niacin, pantothenic acid, vitamin B12, biotin, choline, potassium, magnesium, phosphorus, chloride, copper, selenium, fluoride, and any combinations thereof. Any known sources of these nutrients having nutritional uses may be suitable for use in the composition.

The composition such as growing up milk or cereal may optionally contain other substances that may have a beneficial effect on the host such as lactoferrin, nucleotides, nucleosides, immunoglobulins, CMP equivalents (cytidine 5'-monophosphate, free acid), UMP equivalents (uridine 5'-monophosphate, disodium salt), AMP equivalents (adenosine 5'-monophosphate, free acid), GMP equivalents (guanosine 5'-monophosphate, disodium salt) and combinations thereof.

### Viscosity

The composition comprising non-digestible oligosaccharides may have a viscosity of between 150 and 100,000 mPas at 20 °C and at a shear rate of 10 s-1, such as between 250 and 25,000 mPas, such as between 300 and 10,000 mPas, such as between 500 and 10,000 mPas such as between 1000 and 10,000 mPas. The composition may have a semi-liquid and/or semi-solid constitution. Solid food is still inappropriate for infants changing from breast milk or infant liquid, because of the infant's lack of teeth and its poor swallowing reflex. Semi-liquid may refer to food products that have a viscosity above 150 mPas, but are still pourable. Semi-solid may refer to products that are still formable or spreadable but not pourable, with a viscosity up to 100,000 mPas.

Unless specified otherwise, whenever the term viscosity is used in this document, this refers to the physical parameter which is determined according to the following method: Shear flow viscosities were determined in a Paar Physika MCR 300 Modular Compact Rheometer. The instrument was equipped with a concentric cylinder geometry with a diameter of 27 mm. A logarithmic shear rate ramp is used from 0.1 to 1000 s⁻¹ in 20 minutes having 40 measurement points. Using the same geometry viscosities can also be measured in shear flow at a constant shear rate of 10 s⁻¹ for 10 minutes. The rheometer's thermostat is set on the appropriate temperature (i.e. 20 °C).

To prevent intestinal discomfort, the osmolarity of the semi-liquid and/or semi-solid may be between 300 and 600 mOsm/l, such as between 400 and 500 mOsm/l.

The composition comprising non-digestible oligosaccharides may be in a ready-to-eat form, in which the liquid is already present. In such a form, the product needs only to be heated before consumption and has a stable viscosity during consumption.

The composition comprising non-digestible oligosaccharides may be in the form of granules, flakes, puffs and/or shreds, such as granules.

### Powder

The non-digestible oligosaccharide composition may be in the form of a powder composition. This may comprise: 10 to 80 wt.% cereal based on dry weight of the powder composition; 1.0 to 30 wt.% fibre based on dry weight of the powder composition; and one or more non-digestible oligosaccharides as described in this document.

Reconstitution of this powder with a liquid (such as water or milk) may yield a composition with a viscosity of between 150 and 100,000 mPas. For example, 10 to 100 g powder may be reconstituted with 140 ml liquid (such as water), such as 14 to 80 g powder, such as 30 to 65 g powder, such as 40 to 60 g is reconstituted with 140 ml liquid. For example, the liquid may have a temperature of 30 - 70 °C upon mixing with the powder.

We describe a packaging containing powder composition, wherein the packaging indicates that the powder composition is to be mixed with a suitable amount of liquid.

The powder may be in an agglomerated and/or granulated form with an average particle size below 2 mm, such as below 1 mm. For example, the composition may comprise milk protein, calcium, lactose and fat. This has the advantage that the dried product can be reconstituted with water instead of milk. Water advantageously is more readily available and less prone to contamination than milk. For example, the fat is of vegetable origin. This has the advantage that a healthier product is obtained than when the dried product is reconstituted with cow's milk comprising more saturated fat.

### Administration

The composition described here is administered to infants, preferably infants who are in the adaptation period to solid food, during the weaning stages, infants and/or toddlers attending day care centres. The infant and/or toddler may have an age between 0 and 6 years, preferably between 0 and 36 months, preferably 6 - 36 months, preferably a human toddler with the age of 12-36 months. It is particularly those toddlers who consume mycotoxin-contaminated cereals and cereal products, or are at increased risk thereof. We also provide for a non-therapeutic method for stimulating the health of an infant and/or toddler, comprising administering a composition comprising a non-digestible oligosaccharide A and/or B to the infant and/or toddler.

We further provide for a non-therapeutic method for stimulating the health in an infant and/or toddler comprising the steps a) admixing i) a nutritionally or pharmaceutically acceptable liquid; and ii) a dry composition, wherein the dry composition comprises a non-digestible oligosaccharide A and/or B, and step b) administering the composition obtained in step a) to an infant and/or toddler.

### EXAMPLES

### Example 1

### Introduction

Mycotoxins are toxic natural secondary metabolites formed by fungi growing on agricultural commodities in the field or during storage. At global level, it is considered that 25% of the world crop production is contaminated by mycotoxins, which may be a risk factor for human health due to their toxic properties and their high stability to heat treatment. One of the most prevalent trichothecene mycotoxins is deoxynivalenol (DON). The aim of this example is to investigate the intestinal effect of DON on whey protein allergy through IL-33 mRNA expression, and the ability of galacto-oligosaccharides (GOS) to inhibit the DON-related effects.

### Materials and Methods

Purified DON (D0156; Sigma-Aldrich, St Luis, Mo, USA) was diluted in absolute ethanol (99.9%) to prepare a 25 mM stock solution and was stored at -20°C. Serial dilutions of mycotoxins were prepared in DMEM medium.

Short-chain galacto-oligosaccharides (enzymatic elongation of lactose with galactose by beta-galactosidase) were obtained from FrieslandCampina Domo (Vivinal scGOS sirop, Zwolle, The Netherlands), which contain oligosaccharides with a degree of polymerisation (dp) of 2 - 8 with approximately 45% GOS, 16% lactose, 14% glucose and 25% water. The 0.5%, 1% and 2% scGOS solutions were made in complete cell culture DMEM. Lactose and glucose (FrieslandCampina Domo, Zwolle, The Netherlands), solutions similar to these fractions in the 2% scGOS syrup as well as lactose 2% were used as negative controls to confirm the GOS-specific effects.

### Animals

Male B6C3F1 mice (n=5/6 per group), 6-7 weeks old (Charles River Laboratories) were housed under controlled conditions in standard laboratory cages and were acclimated to the environment for two weeks. They were provided free access to water and food. All *in vivo* experimental protocols were approved by the local Ethics Committee for Animal Experiments (Reference number: DEC 2012.III.02.012) and were performed under strict governmental and international guidelines on animal experimentation.

### Diets and DON gavage

The experimental AIN-93G-based diets were composed and mixed with scGOS by Research Diet Services (Wijk-bij-Duurstede, The Netherlands). The control diet contained per kg food: 180 g protein (100% soy protein isolate), 592 g carbohydrates and 72 g fat (100% soy oil). For the scGOS diet, the control diet was adapted by adding 1 w/w% scGOS. The diet was checked for DON contamination by standard HPLC analyses with affinity column clean-up based on the method as described by Dombrink-Kurtzman et al. (31) and no DON contamination exceeding the limit of 10 µg/kg feed was detected. The mice were fed a control diet or a diet supplemented with scGOS for 2 weeks before an oral DON gavage at a dose of 25mg/kg body weight in 200 µl sterile PBS. Control mice received 200 µl sterile PBS. Feed was withdrawn from cages 2h before toxin administration. The 25 mg/kg dose represents approximately one-half to one-third of the LD50 for DON in mice. Six hours after DON gavage, the mice were sacrificed by cervical dislocation, blood was obtained by heart puncture and different intestine parts were collected for mRNA isolation and histology. Blood was obtained by heart puncture and collected in MiniCollect Z Serum Sep tubes (Greiner Bio-one) and after 1 h clotted blood samples were centrifuged for 10 min at 14.000 rpm and sera was stored at -20°C.

### Isolation of RNA and qRT-PCR mice intestinal samples

For mRNA studies, the mouse intestine was flushed with cold PBS and separated into different segments. These segments were defined as follows: proximal small intestine (first cm of the proximal part of the jejunum, approximately 2 cm after the stomach), middle small intestine (part of the intestine 7-8 cm after the first cm of the proximal part of the jejunum), distal small intestine (final cm before the ileum-caecum-co Ion junction), caecum and colon. These whole intestinal wall samples (approximately 1 cm) were snap frozen in liquid nitrogen and stored at -80 °C until RNA isolation. 50 mg of each sample was suspended into 350 µl RNA Lysis Buffer with β-mercaptoethanol and homogenized using a TissueLyser (Qiagen, Hilden, Germany) for 1 minute/25 Hz. RNA isolation, cDNA synthesis and qRT-PCR reactions were performed as described in Materials and Methods, in vitro experiments. Primer sequences with corresponding annealing temperatures are listed in table 1.

**Table 1. Primer sequences of mouse genes used for qRT-PCR analysis**

| **Target gene** | **Primer sequence (5'-3')** | | **AT** | **Ref** |
|---|---|---|---|---|
| | **Forward** | **Reverse** | | |
| **IL-33** | GGTGTGGATGGGAAGAAGCTG | GAGGACTTTTTGTGAAGGACG | 61 | NM_133775.2 |

### Immunofluorescence and immunohistochemistry mice intestine

The distal small intestine (5 mice/group) was fixed in 10% neutral buffered formalin and embedded in paraffin as a "Swiss roll" to permit a complete microscopic examination. After paraffin embedding, 5 µm sections were cut (2-3 sections/antibody/animal). These Swiss-rolled paraffin sections were deparaffinized, endogenous peroxidase activity was blocked with 0.3% H2O2 (Merck, Darmstadt, Germany) in methanol for 30 min at room temperature and rehydrated in a graded ethanol series to PBS. For antigen retrieval, the slides were boiled in 10 mM citrate buffer (PH 6.0) for 10 min in a microwave. The slides were cooled down to room temperature, rinsed with PBS (3x) and blocked with 5% serum (Dakocytomation, Glostrup, Denmark) in 1% bovine serum albumin in PBS for 30 min at room temperature. Sections for IL-33 immunohistochemistry were incubated with the primary antibody goat-anti-mouse IL-33 (1:1000, AF3626, R&D Systems, Abingdon, United Kingdom) in 1% serum albumin/PBS overnight at 4°C. The slides were rinsed with PBS (3x) and incubated with rabbit-anti-goat poly-HRP Bright vision (DPVG55HRP, Immunologic, Duiven, The Netherlands) for 30 min at room temperature. The slides were rinsed with PBS (3x), followed by diaminobenzidene (DAB) solution (1 DAB tablet in 20 ml PBS, 6.8 µl H2O2 30%) (D5905, Sigma) for 8-10 min at room temperature. Sections were counterstained with Mayers' hematoxylin (Merck), dehydrated and mounted in Permount (Fisher Scientific). Photomicrographs were taken with an Olympus BX50 microscope equipped with a Leica 320 digital camera.

After deparaffinizing, blocking the endogenous peroxidase activity and antigen retrieval step, the immunofluorescence staining of the primary antibody rabbit-anti-claudin-3 (1:50, 34-1700 Invitrogen, CA, USA) on mice intestine was performed as described in Materials and Methods, *in vitro* experiments. The negative controls lacking the primary antibodies were included and no DAB/immunofluorescence signal was detected.

### Histomorphometric analysis mice intestine

The proximal small intestine and distal small intestine were fixed in 10% neutral buffered formalin and embedded as a "Swiss roll". After paraffin embedding, 5 µm sections were cut and stained with hematoxylin/eosin (H&E) according to standard methods. Photomicrographs were taken with an Olympus BX50 microscope equipped with a Leica DFC 320 digital camera. The morphometric analysis of the sections was performed on 10 randomly selected, well-oriented villi and crypts per animal. A computerized microscope-based image analyzer (Cell^D, Olympus, Europa GmbH, Germany) was used to determine histomorphometric parameters: villus height (measured from the tip of the villus to the villus-crypt junction), crypt depth (measured from the crypt-villus junction to the base of the crypt), villus width, villus surface area (total surface of the villus) and epithelial cell area (villus area minus villus area without epithelial cells. These regions were manually defined for each villi separately.

### Statistical analysis

Experimental results are expressed as mean ± S.E.M. Analyses were performed by using GraphPad Prism (version 5.0) (GraphPad, La Jolla, CA). Differences between groups were statistically determined by using One-way ANOVA, with Bonferroni post-hoc test for in vitro experiments and an unpaired two-tailed student's t-test for murine experiments. Results were considered statistically significant when P< 0.05.

### DON-induced IL-33 mRNA expression and distribution pattern is counteracted by scGOS in distal part of the mouse small intestine

The increase in mRNA expression of the pro-Th2 cytokine IL-33 observed after DON gavage was counteracted by scGOS, since the IL-33 mRNA expression levels in the distal small intestine of the scGOS-fed mice were significantly decreased compared to the DON-treated mice without a scGOS diet (Fig. 1). Subsequently, immunohistochemistry was performed to evaluate the IL-33 production along the distal small intestine of the control and DON- treated mice fed with or without a scGOS diet. The results showed that after DON gavage the IL-33 production was obviously increased in the distal small intestine compared to the control mice, especially observed in the epithelial layer around the villi. This DON-related IL-33 induction was moderated in the distal small intestine of the scGOS-fed mice compared to the mice fed with a control diet, since a lower amount of IL-33-expressing epithelial cells was observed. In contrast, systemic IL-33 measured by ELISA in mice serum was neither affected by DON gavage nor by the diet the mice received (data not shown). In vitro, DON-stimulated Caco-2 cells did not induce increased IL-33 mRNA expression levels or increased IL-33 levels in the supernatant. However, scGOS pretreatment resulted in a decrease in IL-33 supernatant levels compared to the unstimulated cells as well as to the DON stimulated cells (data not shown).

### Discussion

The mRNA expression levels of the pro-th2 cytokine IL-33 were upregulated along the intestine, especially in the distal part of the small intestine of the DON-exposed mice compared to the control mice. The increased IL-33 mRNA expression levels in the distal small intestine of the DON-exposed mice were significantly counteracted by scGOS in the diet. To confirm the scGOS-related IL-33 effect in the intestine, the IL-33 distribution along the distal small intestine was evaluated by immunohistochemistry. Interestingly, after DON gavage the IL-33 production was obviously increased in the distal small intestine compared to the control mice, especially observed in the epithelial cells located at the base of the villi. This DON-related IL-33 induction was moderated in the distal small intestine of the scGOS-fed mice compared to the mice fed with a control diet, since a lower amount of IL-33-expressing epithelial cells was observed. In contrast, systemic IL-33 in mice serum was not affected neither by the DON gavage nor by the diet the mice received.

There is a growing body of evidence that IL-33 could possibly play as a key role in mucosal immunity rather than being involved in systemic immune responses, see Ohno (2012)). The effect of non-digestible oligosaccharides on IL-33 mRNA expression and production was never investigated before. IL-33 has been shown to possess potent proinflammatory activity, inducing Th2 cytokine production and promoting Th2 immunity. The most pronounced cytokine effects induced by DON and related to the intervention with scGOS were observed along the distal part of the small intestine. Since DON is quickly and expeditiously absorbed in the upper parts of the small intestine and following absorption it is likely secreted into the gut lumen as DON is a substrate for the efflux transporters ABCB1 (Pg-p) and ABCC2 (MRP2), the distal small intestine is the most susceptible interface in the intestine and, most probably DON exposure will take place from both luminal and basolateral side in the distal part.

### Example 2. Allergic sensitization in an in vivo mouse study

Female C3H/HeOuJ mice were sensitized with a low or high dose deoxynivalenol (DON) with or without the whey protein (20 mg whey) five times weekly, as depicted in Table 2 below. The low dose consisted of 5 times 1 mg/kg body weight, whereas the high dose group received two times 10 mg/kg and three times 5 mg/kg DON. The average body weight of the mice throughout the experiment was 20 gram. At day 24, sera are collected to analyze the allergen-specific antibodies. At day 35, the antigen is intradermal injected (10 µg whey /20 µl PBS/ear) in the ear pinnae and the corresponding ear swelling is measured as a read-out for the local activation of mast cells. The serum collected 30 minutes after the subsequent oral challenge (50 mg whey/0.5 ml PBS/mouse) will give information about the mucosal mast cell response by measuring the mMCP-1. At day 36, the animals are euthanized, and blood and immunological relevant organs isolated.

**Table 2. Overview experimental groups in allergic sensitization study**

| **group** | **n=** | **Sensitization** | | **challenge** |
|---|---|---|---|---|
| | | antigen | adjuvant | |
| A(low DON-PBS) | 4 | vehicle | 20 µg DON | whey |
| B (high DON-PBS) | 4 | vehicle | 200 µg | whey |
| C (low DON-whey) | 7 | 20 mg whey | 20 µg DON | whey |
| D (high DON-whey) | 7 | 20 mg whey | 200 µg | whey |
| E (CT-whey) | 7 | 20 mg whey | 10 µg CT | whey |

The results are plotted in Figure 2. Based on these experiments, it was thus concluded that DON significantly increases whey protein allergy response, particularly in the presence of a food allergen.

It is expected that clinical trials involving scGOS will repeat the above findings found in the *in vitro* studies presented in Figure 1.

### Example 3. Composition according to the invention

| **INGREDIENTS** | **(per 100 kg powder)** |
|---|---|
| Demineralized whey [kg] | 19.4 |
| Vegetable oils [kg] | 19.1 |
| Skimmed milk [kg] | 16.3 |
| Maltodextrin [kg] | 13.5 |
| Dietary fibers [kg]: | 12.7 |
| - galactooligosaccharides [kg] | 11.9 |
| - fructopolysaccharides [kg] | 0.798 |
| Lactose [kg] | 12.6 |
| Whey protein concentrate [kg] | 3.58 |
| Tricalcium phosphate [kg] | 0.398 |
| Fish oil [kg] | 0.384 |
| Calcium carbonate [kg] | 0.356 |
| Tri potassium citrate [kg] | 0.257 |
| Tri sodium citrate [kg] | 0.140 |
| L-ascorbic acid [g] | 87.4 |
| Magnesium chloride[g] | 66.8 |
| Soy lecithin[g] | 59.5 |
| Taurine[g] | 34.1 |
| Choline chloride[g] | 30.1 |
| Vanillin[g] | 30.0 |
| Sodium L-ascorbate[g] | 29.9 |
| Ferrous sulphate[g] | 23.8 |
| Potassium chloride[g] | 23.0 |
| Zinc sulphate[g] | 13.9 |
| DL-alpha tocopheryl acetate[g] | 3.62 |
| Nicotinamide[g] | 2.87 |
| Folic acid[g] | 1.21 |
| Cholecalciferol[g] | 1.19 |
| Calcium D-pantothenate[g] | 1.08 |
| Cupric sulphate[g] | 1.07 |
| Retinyl palmitate[g] | 0.879 |
| DL-alpha tocopherol[g] | 0.836 |
| D-biotin[g] | 0.638 |
| Retinyl acetate[g] | 0.627 |
| Thiamin hydrochloride[g] | 0.402 |
| Cyanocobalamin[g] | 0.325 |
| Pyridoxine hydrochloride[g] | 0.245 |
| Riboflavin[g] | 0.226 |
| Potassium iodide[mg] | 67.0 |
| Manganese sulphate[mg] | 54.6 |
| Phytomenadione[mg] | 27.4 |
| Sodium selenite[mg] | 20.3 |

The fatty acid composition :

| | | |
|---|---|---|
| | Per 100 g fatty acids | Per 100 g powder |
| C16:0 palmitic acid | 19.2 | 3.62 |
| C18:0 stearic acid | 3.50 | 0.66 |
| C18:1 oleic acid | 56.3 | 10.7 |
| C18:2 linoleic acid | 14.0 | 2.65 |
| C18:3 alpha-linolenic acid | 2.60 | 0.492 |
| C20:0 arachidic acid | 0.42 | 0.08 |
| C20:1 eicosaenoic acid | 0.268 | 0.051 |
| C20:4 arachidonic acid | 0.034 | 0.006 |
| C20:5 eicosapentaenoic acid | 0.268 | 0.051 |
| C22:0 behenic acid | 0.44 | 0.08 |
| C22:1 erucic acid | 0.11 | 0.02 |
| C22:5 docosapentaenoic acid | 0.06 | 0.01 |
| C22:6 docosahexaenoic acid | 0.402 | 0.076 |

### Example 4. Effect of scGOS on whey allergy associated with mycotoxin exposure

Female C3H/HeOuJ mice (n=8 per group) mice received a control diet or a diet comprising short-chain galacto-oligosaccharides (said diet containing scGOS/lcFOS (9:1), commercially available as Vivinal®GOS (Borculo Domo Ingredients, Zwolle, Netherlands) and Raftiline (Orafti), respectively). After a run-in period of two weeks, the mice either received an oral exposure to DON plus whey once a week for 5 weeks, or only DON in PBS. Acute allergic skin responses, change in body temperature and other anaphylactic shock reactions were measured upon whey-challenge. Allergen-specific antibodies, Th-2 associated cytokines and ST2 were measured in serum.

Figure 3 reports the allergic reaction effects for the compositions comprising abudant levels of galacto-oligosaccharides, and it showed advantageous effects in all events.

## Claims

1. A composition for use in reducing the risk of occurrence of or preventing whey protein allergy associated with mycotoxin exposure in an infant who consumes cereals or cereal-comprising products daily and suffers from an increased risk of whey protein allergy associated with mycotoxin exposure, said composition comprising non-digestible oligosaccharide.

2. The composition for use according to claim 1, wherein said infant is at increased risk of trichothecene mycotoxin exposure, and wherein said infant is preferably at increased risk of exposure to the food allergen.

3. The composition for use according to any of the preceding claims, wherein said infant suffers from an increased risk of trichothecene mycotoxin exposure associated whey protein allergy, preferably deoxynivalenol exposure associated whey protein allergy.

4. The composition for use according to any of the preceding claims, wherein the non-digestible oligosaccharide has a degree of polymerisation (DP) of 2 - 10.

5. The composition for use according to claim 4, wherein the non-digestible oligosaccharide comprises a galactooligosaccharide and/or a fructooligosaccharide, preferably at least a galactooligosaccharide, more preferably at least transgalactooligosaccharide.

6. The composition for use according to any of the preceding claims, wherein the non-digestible oligosaccharide comprises a short-chain galactooligosaccharide and/or a short-chain fructooligosaccharide, preferably at least both.

7. The composition for use according to any of the preceding claims, wherein said infant is a baby or weaning infant preferably of at least 6 months of age, or a weaning toddler, preferably a toddler between 12 to 36 months old.

8. The composition for use according to any of the preceding claims, wherein said infant is a toddler who consumes mycotoxin-contaminated cereals and cereal products, or is at increased risk thereof.

9. A non-therapeutic method for providing nutrition to an infant who consumes cereals or cereal-comprising products daily and suffers from an increased risk of whey protein allergy associated with mycotoxin exposure, comprising administering non-digestible oligosaccharides to said infant.

10. The non-therapeutic method according to claim 9, wherein said infant suffers from an increased risk of trichothecene mycotoxin exposure associated whey protein allergy, preferably deoxynivalenol exposure associated whey protein allergy.

11. The non-therapeutic method according to claim 9 or 10, wherein the non-digestible oligosaccharide has a degree of polymerisation (DP) of 2 - 10.

12. The non-therapeutic method according to any one of claim 9 - 11, wherein the non-digestible oligosaccharide comprises a galactooligosaccharide and/or a fructooligosaccharide, preferably at least a galactooligosaccharide, more preferably at least transgalactooligosaccharide.

13. The non-therapeutic method according to any one of claim 9 - 12, wherein the non-digestible oligosaccharide comprises a short-chain galactooligosaccharide and/or a short-chain fructooligosaccharide, preferably at least both.

14. The non-therapeutic method according to any of claims 9 - 13, wherein said infant is a toddler who consumes mycotoxin-contaminated cereals and cereal products, or is at increased risk thereof.

15. A composition for use in reducing the risk of occurrence of or preventing food allergy associated with mycotoxin exposure, atopic dermatitis associated with mycotoxin exposure or skin allergy associated with mycotoxin exposure in an infant who consumes cereals or cereal-comprising products daily and suffers from an increased risk of food allergy associated with mycotoxin exposure, atopic dermatitis associated with mycotoxin exposure or skin allergy associated with mycotoxin exposure, said composition comprising non-digestible oligosaccharide.

## Patentansprüche

1. Zusammensetzung zur Verwendung bei der Verringerung des Risikos des Auftretens oder Verhinderung von mit Mykotoxinexposition assoziierter Molkeproteinallergie bei einem Kind, welches täglich Getreide oder Getreide-umfassende Produkte konsumiert und von einem erhöhten Risiko zur mit Mykotoxinexposition assoziierter Molkeproteinallergie betroffen ist, wobei besagte Zusammensetzung nichtverdauliche Oligosaccharide umfasst.

2. Zusammensetzung zur Verwendung nach Anspruch 1, wobei besagtes Kind ein erhöhtes Risiko zur Trichothecen-Mykotoxinexposition hat, und wobei besagtes Kind bevorzugt ein erhöhtes Risiko zur Exposition mit dem Lebensmittelallergen hat.

3. Zusammensetzung zur Verwendung nach einem der vorangehenden Ansprüche, wobei besagtes Kind von einem erhöhten Risiko zur mit Trichothecen-Mykotoxinexposition assoziierten Molkeproteinallergie betroffen ist, bevorzugt mit Deoxynivalenolexposition assoziierter Molkeproteinallergie.

4. Zusammensetzung zur Verwendung nach einem der vorangehenden Ansprüche, wobei das nichtverdauliche Oligosaccharid einen Polymerisationsgrad (DP) von 2 - 10 hat.

5. Zusammensetzung zur Verwendung nach Anspruch 4, wobei das nichtverdauliche Oligosaccharid ein Galactooligosaccharid und/oder ein Fructooligosaccharid umfasst, bevorzugt mindestens ein Galactooligosaccharid, bevorzugter mindestens Transgalactooligosaccharid.

6. Zusammensetzung zur Verwendung nach einem der vorangehenden Ansprüche, wobei das nichtverdauliche Oligosaccharid ein kurzkettiges Galactooligosaccharid und/oder ein kurzkettiges Fructooligosaccharid umfasst, bevorzugt mindestens beide.

7. Zusammensetzung zur Verwendung nach einem der vorangehenden Ansprüche, wobei besagtes Kind ein Säugling oder abgestilltes Kind ist, bevorzugt im Alter von mindestens 6 Monaten, oder ein abgestilltes Kleinkind, bevorzugt ein Kleinkind im Alter zwischen 12 und 36 Monaten.

8. Zusammensetzung zur Verwendung nach einem der vorangehenden Ansprüche, wobei besagtes Kind ein Kleinkind ist, welches Mykotoxin-kontaminiertes Getreide und Getreideprodukte konsumiert oder ein erhöhtes Risiko dazu hat.

9. Nichttherapeutisches Verfahren zur Ernährung eines Kindes, welches täglich Getreide oder Getreide-umfassende Produkte konsumiert und von einem erhöhten Risiko zur mit Mykotoxinexposition assoziierter Molkeproteinallergie betroffen ist, welches die Verabreichung nichtverdaulicher Oligosaccharide an besagtes Kind umfasst.

10. Nichttherapeutisches Verfahren nach Anspruch 9, wobei besagtes Kind von einem erhöhten Risiko zur mit Trichothecen-Mykotoxinexposition assoziierter Molkeproteinallergie betroffen ist, bevorzugt Deoxynivalenolexposition assoziierte Molkeproteinallergie.

11. Nichttherapeutisches Verfahren nach Anspruch 9 oder 10, wobei das nichtverdauliche Oligosaccharid einen Polymerisationsgrad (DP) von 2 - 10 hat.

12. Nichttherapeutisches Verfahren nach einem der Ansprüche 9 - 11, wobei das nichtverdauliche Oligosaccharide ein Galactooligosaccharid und/oder ein Fructooligosaccharid umfasst, bevorzugt mindestens ein Galactooligosaccharid, bevorzugter mindestens Transgalactooligosaccharid.

13. Nichttherapeutisches Verfahren nach einem der Ansprüche 9 - 12, wobei das nichtverdauliche Oligosaccharide ein kurzkettiges Galactooligosaccharid und/oder ein kurzkettiges Fructooligosaccharid umfasst, bevorzugt mindestens beide.

14. Nichttherapeutisches Verfahren nach einem der Ansprüche 9 - 13, wobei besagtes Kind ein Kleinkind ist, welches Mykotoxin-kontaminiertes Getreide und Getreideprodukte konsumiert oder ein erhöhtes Risiko dazu hat.

15. Zusammensetzung zur Verwendung bei der Verringerung des Risikos des Auftretens oder Verhinderung von mit Mykotoxinexposition assoziierter Molkeproteinallergie, mit Mykotoxinexposition assoziierter atopischer Dermatitis oder mit Mykotoxinexposition assoziierter Hautallergie bei einem Kind, welches täglich Getreide oder Getreide-umfassende Produkte konsumiert und von einem erhöhten Risiko zur mit Mykotoxinexposition assoziierter Lebensmittelallergie, mit Mykotoxinexposition assoziierte atopischer Dermatitis oder mit Mykotoxinexposition assoziierter Hautallergie betroffen ist, wobei besagte Zusammensetzung nichtverdauliche Oligosaccharide umfasst.

## Revendications

1. Composition destinée être utilisée pour réduire le risque d'apparition ou pour empêcher une allergie aux protéines de lactosérum associée à une exposition aux mycotoxines chez un nourrisson qui consomme quotidiennement des céréales ou des produits céréaliers et présente un risque accru d'allergie aux protéines de lactosérum associée à une exposition aux mycotoxines, ladite composition comprenant un oligosaccharide non digestible.

2. Composition destinée être utilisée selon la revendication 1, dans laquelle ledit nourrisson présente un risque accru d'exposition aux mycotoxines trichothécènes, et dans lequel ledit nourrisson présente de préférence un risque accru d'exposition à l'allergène alimentaire.

3. Composition destinée être utilisée selon l'une quelconque des revendications précédentes, dans laquelle ledit nourrisson présente un risque accru d'exposition aux mycotoxines trichothécènes associées à une allergie aux protéines de lactosérum, de préférence une exposition au déoxynivalénol associé à une allergie aux protéines de lactosérum.

4. Composition destinée être utilisée selon l'une quelconque des revendications précédentes, dans laquelle l'oligosaccharide non digestible a un degré de polymérisation (DP) de 2 à 10.

5. Composition destinée être utilisée selon la revendication 4, dans laquelle l'oligosaccharide non digestible comprend un galactooligosaccharide et/ou un fructooligosaccharide, de préférence au moins un galactooligosaccharide, de manière plus préférée au moins un transgalactooligosaccharide.

6. Composition destinée être utilisée selon l'une quelconque des revendications précédentes, dans laquelle l'oligosaccharide non digestible comprend un galactooligosaccharide à chaîne courte et/ou un fructooligosaccharide à chaîne courte, de préférence au moins les deux.

7. Composition destinée être utilisée selon l'une quelconque des revendications précédentes, dans laquelle ledit nourrisson est un bébé ou un nourrisson âgé de préférence d'au moins 6 mois, ou un jeune enfant en âge de sevrage, de préférence un jeune enfant âgé de 12 à 36 mois.

8. Composition destinée être utilisée selon l'une quelconque des revendications précédentes, dans laquelle ledit nourrisson est un jeune enfant qui consomme des céréales et des produits céréaliers contaminés par des mycotoxines, ou qui en présente un risque accru.

9. Procédé non thérapeutique pour fournir une nutrition à un nourrisson qui consomme des céréales ou des produits contenant des céréales quotidiennement et qui présente un risque accru d'allergie aux protéines de lactosérum associée à une exposition aux mycotoxines, comprenant l'administration d'oligosaccharides non digestibles audit nourrisson.

10. Procédé non thérapeutique selon la revendication 9, dans lequel ledit nourrisson présente un risque accru d'exposition aux mycotoxines trichothécènes associées à une allergie aux protéines de lactosérum, de préférence une exposition au déoxynivalénol associé à une allergie aux protéines de lactosérum.

11. Procédé non thérapeutique selon la revendication 9 ou 10, dans lequel l'oligosaccharide non digestible a un degré de polymérisation (DP) de 2 à 10.

12. Procédé non thérapeutique selon l'une quelconque des revendications 9 à 11, dans lequel l'oligosaccharide non digestible comprend un galactooligosaccharide et/ou un fructooligosaccharide, de préférence au moins un galactooligosaccharide, de manière plus préférée au moins un transgalactooligosaccharide.

13. Procédé non thérapeutique selon l'une quelconque des revendications 9 à 12, dans lequel l'oligosaccharide non digestible comprend un galactooligosaccharide à chaîne courte et/ou un fructooligosaccharide à chaîne courte, de préférence au moins les deux.

14. Procédé non thérapeutique selon l'une quelconque des revendications 9 à 13, dans lequel ledit nourrisson est un jeune enfant qui consomme des céréales et des produits céréaliers contaminés par des mycotoxines, ou en présente un risque accru.

15. Composition destinée être utilisée pour réduire le risque d'apparition ou pour empêcher une allergie aux protéines de lactosérum associée à une exposition aux mycotoxines, une dermatite atopique associée à une exposition aux mycotoxines ou une allergie cutanée associée à l'exposition aux mycotoxines chez un nourrisson qui consomme quotidiennement des céréales ou des produits céréaliers et présente un risque accru d'allergie alimentaire associée à une exposition aux mycotoxines, d'une dermatite atopique associée à une exposition aux mycotoxines ou d'une allergie cutanée associée à une exposition aux mycotoxines, ladite composition comprenant des oligosaccharides non digestibles.
